# EUROPÄISCHE PATENTSCHRIFT

(11) **EP 2 941 207 B1**
(45) Veröffentlichungstag und Bekanntmachung des Hinweises auf die Patenterteilung: **08.02.2017**
(21) Anmeldenummer: 13830209.6
(22) Anmeldetag: 23.12.2013
(51) Int. Cl.: A61B 17/16

(54) **CHIRURGISCHES SCHIEBESCHAFTINSTRUMENT**
SURGICAL SLIDING SHAFT INSTRUMENT
INSTRUMENT CHIRURGICAL À TIGE COULISSANTE

(30) Priorität: 02.01.2013 DE 102013100021
(43) Veröffentlichungstag der Anmeldung: 11.11.2015
(73) Patentinhaber: Asanus Medizintechnik GmbH, 78579 Neuhausen ob Eck (DE)
(72) Erfinder: SCHORER, Armin, 78597 Irndorf (DE)
(74) Vertreter: CBDL Patentanwälte
(86) Internationale Anmeldenummer: PCT/DE2013/100439
(87) Internationale Veröffentlichungsnummer: WO 2014/106505

(56) Entgegenhaltungen:
- DE-B3-102007 054 432
- DE-U1-202009 009 562
- US-A- 5 961 531

## Beschreibung

### TECHNISCHES GEBIET DER ERFINDUNG

Die Erfindung betrifft ein chirurgisches Schiebeschaftinstrument, mit einem Schaft und einem Schieber, der mittels eines Griffstücks entlang des Schafts bewegt werden kann, wie dies z.B. bei Rongeuren, Conchotomen und Knochenstanzen bekannt ist.

### HINTERGRUND DER ERFINDUNG

Schiebeschaftinstrumente der hier in Frage stehenden Art sind bekannt und dienen in der Regel zum Durchtrennen und/oder Entfernen von Gewebe, Knochen oder Knorpel. Dazu besitzen die Schiebeschaftinstrumente einen Schaft, der proximal in einem ersten Griffstück mündet, und einen Schieber, der mittels eines gelenkig mit dem ersten Griffstück verbundenen zweiten Griffstücks entlang des Schafts bewegt werden kann, um am distalen Ende des Instruments eine Aktion auszulösen, also z.B. bei bestimmten Rongeuren ein Maulteil auf- und zuzuklappen, bei Conchotomen zwei Schneiden gegeneinander zu bewegen oder bei Knochenstanzen eine Stanzform gegen ein Widerlager zu pressen.

Instrumente der hier in Frage stehenden Art müssen nach der Verwendung gründlich gereinigt und sterilisiert werden. Eine besondere Problemzone stellt dabei der Bereich zwischen Schieber und Schaft dar. Es wurden daher verschiedene Instrumente vorgeschlagen, bei denen der Schieber zu Reinigungszwecken zumindest partiell vom Schaft getrennt werden kann.

Aus der DE 10 2007 054 432 B3, der DE 197 02 079 A1 und der DE 197 48 369 A1 sind chirurgische Schiebeschaftinstrumente bekannt, die zur Reinigung komplett zerlegt werden können. Das Zerlegen und wieder Zusammenfügen ist jedoch zeitaufwendig, und es besteht die Gefahr, Einzelteile während des Reinigungsprozesses zu verlieren.

Die DE 20 2009 009 562 U1 zeigt ein Schiebeschaftinstrument gemäß dem Oberbegriff von Anspruch 1, wobei zur Verbindung von Schieber und Schaft ein Verriegelungsmechanismus mit einem Mitnehmer und einer Aufnahme vorgesehen umfasst, wobei der Mitnehmer in eine Arbeitsposition, in der er sich in der Aufnahme befindet und die Aufnahme nicht verlassen kann, und in eine Freigabeposition, in der er die Aufnahme über einen Schlitz verlassen kann, bewegbar ist. Der Mitnehmer ist dabei jedoch mittels einer Feder in die Arbeitsposition vorgespannt. Federbelastete Verriegelungsmechanismen sind jedoch verhältnismäßig aufwendig in der Montage und zudem nicht einfach zu reinigen.

Aus der US 5,961,531 und der DE 299 24 518 U1 sind Schiebeschaftinstrumente bekannt, bei denen der Schieber jeweils nach Lösen eines Verriegelungsmechanismus um eine nahe einer Drehachse, um die zwei Griffstücke zur Bewegung des Schiebers relativ zueinander bewegbar sind, befindliche und zu der genannten Drehachse parallele Schwenkachse schwenkbar ist. Die DE 20 2009 003 869 U1 zeigt ein ähnliches Schiebeschaftinstrument, bei dem jedoch die Schwenkachse, um die der Schieber nach Lösen eines Verriegelungsmechanismus schwenkbar ist, rechtwinklig zu der Drehachse verläuft, um die die Griffstücke zur Bewegung des Schiebers relativ zueinander bewegbar sind. Die bei diesen Schiebeschaftinstrumenten eingesetzten Verriegelungsmechanismen arbeiten teilweise (DE 20 2009 003 869 U1) ebenfalls mit Federn zur Vorspannung eines verriegelnden Elementes in eine bestimmte Stellung oder mehrere eng beieinanderliegend angeordnete bewegliche aber vom Instrument nicht ohne weiteres abnehmbare Teile, was die Montage, insbesondere aber die Reinigung und Sterilisation schwierig macht.

### OFFENBARUNG DER ERFINDUNG

Der Erfindung liegt die Aufgabe zugrunde, ein Schiebeschaftinstrument mit einem besonders einfach aufgebauten Verriegelungsmechanismus anzugeben, der ohne federbelastete Teile auskommt und gleichwohl eine sichere Halterung des Schiebers ermöglicht. Zudem soll der Mechanismus aus möglichst wenigen Teilen bestehen, die zudem zwecks Reinigung gut zugänglich sein sollen.

Die Aufgabe wird gelöst von einem Schiebeschaftinstrument gemäß Anspruch 1. Vorteilhafte Ausgestaltungen und Weiterbildungen sind Gegenstand der Unteransprüche. Das erfindungsgemäße Schiebeschaftinstrument kann so ausgebildet sein, dass der Schieber nach Öffnen des Verriegelungsmechanismus um eine im Bereich seines distalen Endes vorgesehene Achse vom Schaft und von Griffstücken weggeschwenkt werden kann, dabei aber fest mit dem Schaft verbunden bleibt, so dass das Instrument gut gereinigt werden kann, gleichzeitig aber die Gefahr minimiert ist, bei der Reinigung und insbesondere der Sterilisation, die in entsprechenden Maschinen erfolgen, Teile des Instruments zu verlieren oder Teile verschiedener Instrumente miteinander zu vertauschen, was aus Qualitätssicherungsgründen (um z.B. die Lebensdauer eines Instruments genau zu überwachen) selbst dann nicht erwünscht ist, wenn baugleiche Teile miteinander vertauscht werden,

Weitere Einzelheiten und Vorteile der Erfindung ergeben sich aus der nachfolgenden rein beispielhaften und nicht-beschränkenden Beschreibung eines Ausführungsbeispiels in Verbindung mit der Zeichnung.

### KURZE BESCHREIBUNG DER ZEICHNUNG

- Fig. 1: zeigt eine Seitenansicht eines erfindungsgemäßen Schiebeschaftinstruments in einer Arbeitsstellung.
- Fig. 2: zeigt eine Seitenansicht des Schiebeschaftinstruments gemäß Fig. 1 in einer Reinigungsstellung.
- Fig. 3: zeigt die Aufnahme für den Mitnehmer bei dem in der Reinigungsstellung gemäß Fig. 2 befindlichen Schiebeschaftinstrument in vergrößertem Maßstab.
- Fig. 4: zeigt einen Blick auf einen Teil derjenigen Seite des Schiebers, die in der Arbeitsstellung am Schaft anliegt.
- Fig. 5: zeigt einen Abschnitt des Hebels zur Verstellung des Mitnehmers.
- Fig. 6: zeigt einen Schnitt durch den Hebel entlang Linie VI-VI in Fig. 5.
- Fig. 7: zeigt eine Seitenansicht eines Teilabschnitts eines erfindungsgemäßen Schiebeschaftinstruments, bei dem in einem der Griffstücke eine Führungsrinne für einen Rastvorsprung des zur Verstellung des Mitnehmers dienenden Hebels vorgesehen ist.

### BESCHREIBUNG BEVORZUGTER AUSFÜHRUNGSFORMEN

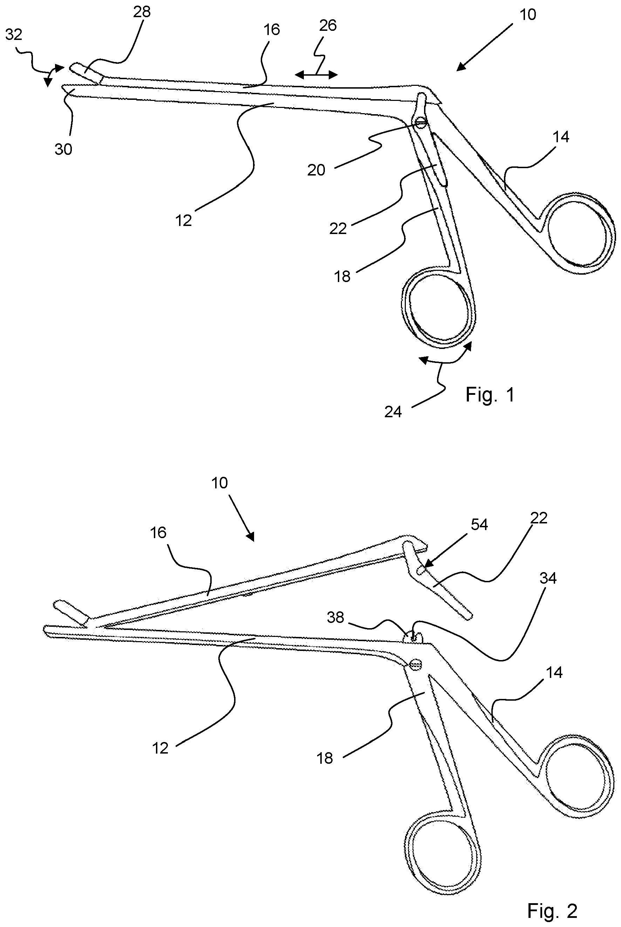

In der Fig. 1 ist ein in seiner Gesamtheit mit 10 bezeichnetes Schiebeschaftinstrument gezeigt, das einen Schaft 12, der proximal in einem ersten Griffstück 14 mündet, und einen Schieber 16 aufweist, der mittels eines gelenkig mit dem ersten Griffstück 14 verbundenen zweiten Griffstücks 18 entlang des Schafts 12 bewegt werden kann. Zur Verbindung der beiden Griffstücke dient bei diesem Ausführungsbeispiel ein Drehbolzen 20, der zudem vorteilhaft eine Doppelfunktion erfüllt und auch als Anschlag und Widerlager für einen schwenkbaren Hebel 22 dient.

Eine durch den Bewegungspfeil 24 angedeutete Schwenkbewegung des Griffstücks 18 wird in an sich bekannter Weise in eine durch den Bewegungspfeil 26 angedeutete translatorische Bewegung des Schiebers 16 entlang des Schaftes 12 umgesetzt. Üblicherweise wird das gezeigte Instrument so gegriffen, das der Daumen in die Grifföffnung des Griffstücks 14 und der Zeige- oder Mittelfinger in die Grifföffnung des Griffstücks 18 eingeführt wird. Bei Benutzung werden dann das Instrument mittels des Daumens in Position gehalten und der Schaft mittels des Zeige- und/oder Mittelfingers bewegt. Das erfindungsgemäße Instrument kann jedoch in einfacher Weise auch so ausgestaltet werden, dass bei Benutzung das Instrument mittels des Zeige- und/oder Mittelfingers in Position gehalten und der Schaft mittels des Daumens bewegt werden, wozu die Anordnung von erstem und zweitem Griffstück quasi vertauscht werden muss. Mit Blick auf zum Beispiel Fig. 1 kann man sagen, dass bei dem gezeigten Ausführungsbeispiel der Schaft 12 mit dem ersten Griffstück 14 eine erste gedachte Linie bildet, wobei die Linie aus zwei Teilstücken, eben dem Schaft und dem Griffstück besteht, die hier einen Winkel von etwa 135° einschließen.

Der Schieber 16 bildet mit dem zweiten Griffstücks 18 eine zweite gedachte Linie, ebenfalls aus zwei Teilstücken, die in der in Fig. 1 gezeigten Stellung einen Winkel von etwa 105° einschließen, wobei das Griffstück 18 relativ zum Schieber 16 beweglich und daher der von dem Griffstück 18 und dem Schieber 16 eingeschlossene Winkel veränderlich ist. Die beiden genannten gedachten abgewinkelten Linien kreuzen sich bei dem gezeigten Ausführungsbeispiel, so dass bei natürlicher Griffhaltung das erste Griffstück 14 mit dem Daumen und das zweite Griffstück 18 mit dem Zeige- und/oder Mittelfinger gehalten werden. Das zweite Griffstück kann jedoch auch so am ersten Griffstück angelenkt werden, dass, wenn wiederum der Schaft mit dem ersten Griffstück eine erste gedachte Linie und der Schieber mit dem zweiten Griffstücks eine zweite Linie bilden, die beiden Linien einander nicht kreuzen, so dass bei natürlicher Griffhaltung das erste Griffstück mit dem Zeige- und/oder Mittelfinger und das zweite Griffstück mit dem Daumen gehalten werden, was die Handhabung vorteilhaft erleichtern kann, da es von vielen Personen als einfacher empfunden wird, ein Instrument mittels Zeige- und/oder Mittelfinger in Position zu halten und dann lediglich den Daumen zu bewegen.

Das Schiebeschaftinstrument ist bei diesem Ausführungsbeispiel ein in Deutschland üblicherweise als Rongeur bezeichnetes Instrument, bei dem am distalen Ende zwei Maulteile 28 und 30 mit Schneidkanten ausgebildet sind, von denen eines, hier das Maulteil 28, in ebenfalls an sich bekannter Weise über ein Gelenkstück, an dem auch das distale Ende des Schiebers 16 angelenkt ist, beweglich mit dem Schaft 12 verbunden ist, während das andere Maulteil 30 das distale Ende des Schaftes 12 bildet. Das Gelenkstück erlaubt auch eine Schwenkbewegung des Schiebers derart, dass sein proximales Ende wie in Fig. 2 gezeigt vom Schaft 12 weggeschwenkt werden kann, wobei vorteilhaft alle Teile des Instruments miteinander verbunden bleiben, so dass sie zwar leicht gereinigt, aber nicht verloren oder vertauscht werden können.

Eine translatorische Bewegung des Schiebers 16 entlang des Schaftes 12 bewirkt bei diesem Ausführungsbeispiel ein Auf- und Zuklappen des Maulteils 28, wie durch den Bewegungspfeil 32 angedeutet. Es sei bemerkt, das in anderen Ländern auch anders ausgebildete Schiebeschaftinstrumente, z.B. die in Deutschland als Knochenstanzen bezeichneten Instrumente, als Rongeure bezeichnet werden. Die Erfindung ist aber bei Schiebeschaftinstrumenten beliebiger Art anwendbar.

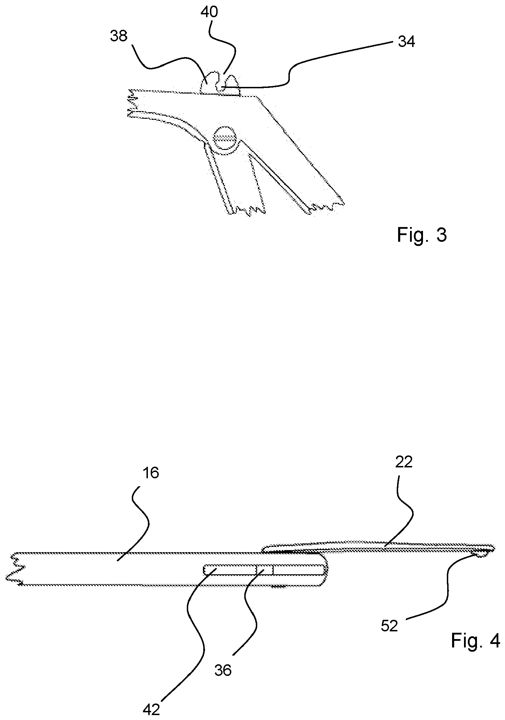

Wie aus den Fig. 2 bis 6 verständlich, sind der Schieber 16 und das zweite Griffstück 18 über einen Verriegelungsmechanismus lösbar miteinander verbunden, wobei der Verriegelungsmechanismus eine Aufnahme 34 und einen Mitnehmer 36 (sichtbar z.B. in Fig. 4) aufweist.

Die Aufnahme 34 ist bei diesem Ausführungsbeispiel in einer Lasche 38 ausgebildet und mit einem Schlitz 40 versehen, über den der Mitnehmer 36 in die Aufnahme 32 eingeführt werden kann. Die Lasche setzt das zweite Griffstück 18 fort und durchdringt den Schaft 12 im Bereich des Übergangs von Schaft und erstem Griffstück 14, wozu der Schaft entsprechend ausgenommen ist.

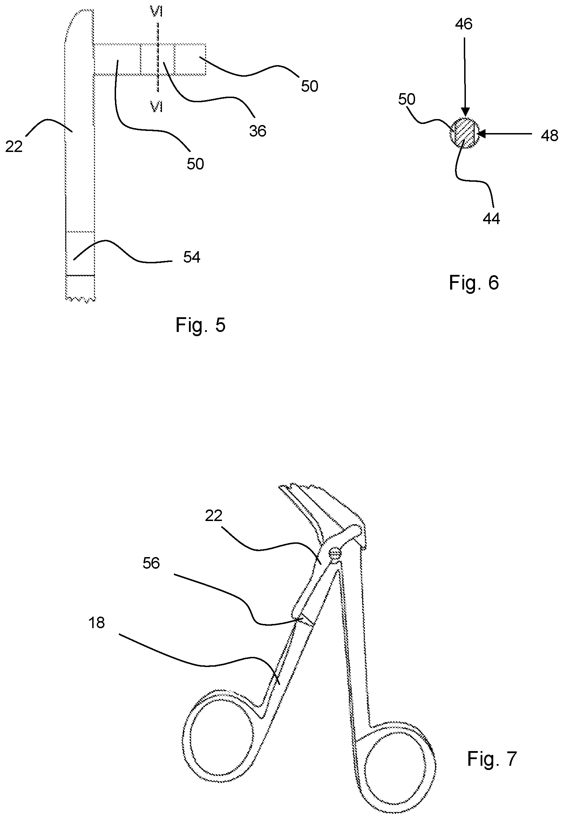

Der Mitnehmer 36 kann in eine Arbeitsposition, in der er sich in der Aufnahme 34 befindet und die Aufnahme 34 nicht verlassen kann, und eine Freigabeposition, in der er die Aufnahme 34 über den Schlitz 40 verlassen kann, geschwenkt werden, und zwar mittels des Hebels 22. Dazu ist der Mitnehmer 36, wie in Fig. 5 gut zu erkennen, vom Hebel 22 rechtwinklig abstehend ausgebildet. Der Mitnehmer 36 ist quer zur Längsrichtung des Schiebers 16 durch den Schieber 16 hindurchgeführt, wobei der Schieber 16 im Bereich des Mitnehmers 36 eine Aussparung 42 für die Lasche 38 aufweist.

Der bei diesem Ausführungsbeispiel nahezu quaderförmige Mitnehmer 36 besitzt zwei schmalen Seiten 46 und zwei breiten Seiten 48 (vgl. Fig. 6), die so bemessen sind, dass der Mittelteil nur mit einer seiner beiden schmalen Seiten 46 voran durch den Schlitz 40 passt, nicht aber mit einer seiner breiten Seiten 48 voran. Mittels des Hebels 22 kann der Mitnehmer dann in eine Freigabeposition, in der eine schmale Seite 46 des Mitnehmers 36 zum Schlitz 40 weist, so dass der Mitnehmer 36 in die Aufnahme 34 hinein bzw. aus dieser heraus geführt werden kann, und in eine Arbeitsposition, in der eine breite Seite 48 des Mitnehmers 36 zum Schlitz 40 weist, so dass der Mitnehmer 36 die Aufnahme 34 nicht durch den Schlitz 40 verlassen kann, gedreht werden.

Wie in den Fig. 5 und 6 zu erkennen, ist der Mitnehmer 36 bei diesem Ausführungsbeispiel an zwei einander gegenüberliegenden Enden von zwei zylindrischen Endstücken 50 eingefasst. Der Außendurchmesser dieser Endstücke 50 ist an den Innendurchmesser einer im Schieber 16 vorgesehenen Aufnahmebohrung angepasst ist, so dass der Hebel mit dem Mitnehmer genau in den Schieber eingepasst werden kann und sich keine Zwischenräume bilden, in die Blut oder andere Körperflüssigkeiten eindringen könnten..

Der Mitnehmer 36 ist in der Arbeitsposition verriegelbar. Dies wird bei diesem Ausführungsbeispiel dadurch bewirkt, dass der Hebel 22 mit einem Rastvorsprung 52 versehen ist, so dass der Hebel 22 in der Arbeitsposition an dem Griffstück 14 verrastet, wobei er sich an dem Drehbolzen 20, wie oben bereits erwähnt, abstützt. Dazu ist der Hebel mit einer Ausnehmung 54 für den Drehbolzen 20 versehen.

Um ein leichtes Überführen des Hebels 22 mit dem Rastvorsprung 52 in die Raststellung zu ermöglichen, kann, wie in Fig. 7 gezeigt, an dem Griffstück 18, an dem der Hebel 22 in der Arbeitsposition des Mitnehmers 36 verrastet, eine Führungsrinne 56 für den Rastvorsprung 52 vorgesehen ist.

Im Rahmen des Erfindungsgedankens sind zahlreiche Abwandlungen und Weiterbildungen möglich, die sich z.B. auf die Gelenkmechanismen zwischen erstem und zweitem Griffstück, die Anlenkung des Schiebers und die Verriegelung des Mitnehmers in der Arbeitsposition beziehen. Auch ist es möglich, erstes und zweites Griffstück gewissermaßen zu vertauschen, so dass der Schaft zwar immer noch im ersten Griffstück mündet, dieses aber bei natürlicher Griffhaltung mit einem Zeige- und/oder Mittelfinger gegriffen wird, während das zweite Griffstück und damit der Schieber mittels des Daumens bewegt werden.

### BEZUGSZEICHENLISTE

- 10: Schiebeschaftinstrument
- 12: Schaft
- 14: erstes Griffstück
- 16: Schieber
- 18: zweites Griffstück
- 20: Drehbolzen, zugleich Anschlag für Hebel
- 22: Hebel
- 24: Bewegungspfeil
- 26: Bewegungspfeil
- 28: Maulteil
- 30: Maulteil
- 32: Bewegungspfeil
- 34: Aufnahme
- 36: Mitnehmer
- 38: Lasche
- 40: Schlitz
- 42: Aussparung
- 46: schmale Seite
- 48: breite Seite
- 50: zylindrisches Endstück
- 52: Rastvorsprung
- 54: Ausnehmung
- 56: Führungsrinne

## Patentansprüche

1. Chirurgisches Schiebeschaftinstrument (10) mit
- einem Schaft (12), der proximal in einem ersten Griffstück (14) mündet, und
- einem Schieber (16), der mittels eines gelenkig mit dem ersten Griffstück (14) verbundenen zweiten Griffstücks (18) entlang des Schafts (12) bewegt werden kann,
- wobei der Schieber (16) und das zweite Griffstück (18) über einen Verriegelungsmechanismus lösbar miteinander verbunden sind,
- wobei der Verriegelungsmechanismus einen Mitnehmer (36) und eine Aufnahme (34) aufweist und
- wobei die Aufnahme (34) einen Schlitz (40) für den Mitnehmer (36) aufweist und der Mitnehmer (36) in eine Arbeitsposition, in der er in der Aufnahme (34) gehalten ist, und eine Freigabeposition, in der er die Aufnahme (34) über den Schlitz (40) verlassen kann, bewegbar ist,
- wobei der Mitnehmer (36) in die Arbeitsposition und die Freigabeposition mittels eines Hebels (22) schwenkbar ist,
- wobei der Mitnehmer (36) in der Arbeitsposition verriegelbar ist,
- wobei der Hebel (22) mit einem Rastvorsprung (52) versehen ist,
**dadurch gekennzeichnet,**
- **dass** der Hebel (22) in der Arbeitsposition des Mitnehmers (36) an einem der beiden Griffstücke (18) verrastet.

2. Schiebeschaftinstrument nach Anspruch 1, **dadurch gekennzeichnet, dass** an dem Griffstück (18), an dem der Hebel (22) in der Arbeitsposition des Mitnehmers (36) verrastet, eine Führungsrinne (56) für den Rastvorsprung (52) vorgesehen ist.

3. Schiebeschaftinstrument nach Anspruch 1 oder 2, **dadurch gekennzeichnet, dass** ein Anschlag für den Hebel (22) vorgesehen ist.

4. Schiebeschaftinstrument nach Anspruch 3, **dadurch gekennzeichnet, dass** der Anschlag von einem Gelenkbolzen (20) gebildet wird, der die beiden Griffstücke (14, 18) gelenkig miteinander verbindet.

5. Schiebeschaftinstrument nach einem der Ansprüche 1 bis 4, **dadurch gekennzeichnet, dass** die Aufnahme (34) an dem zweiten Griffstück (18) vorgesehen ist und dass der Mitnehmer (36) quer zur Längsrichtung des Schiebers (16) durch den Schieber (16) geführt ist, wobei der Schieber (16) im Bereich des Mitnehmers (36) eine Aussparung (42) für eine die Aufnahme (34) aufweisende Lasche (38) besitzt.

6. Schiebeschaftinstrument nach einem der Ansprüche 1 bis 5, **dadurch gekennzeichnet, dass** der Mitnehmer (36) wenigstens eine schmale Seite (46) und wenigstens eine breite Seiten (48) aufweist, die so bemessen sind, dass der Mitnehmer nur mit der schmalen Seite (46) voran durch den Schlitz (40) geführt werden kann.

7. Schiebeschaftinstrument nach Anspruch 5 und Anspruch 6, **dadurch gekennzeichnet, dass** der Mitnehmer (36) an zwei einander gegenüberliegenden Enden von zwei zylindrischen Endstücken (50) eingefasst ist, deren Außendurchmesser an den Innendurchmesser einer im Schieber (16) vorgesehenen Aufnahmebohrung angepasst ist.

8. Schiebeschaftinstrument nach einem der Ansprüche 1 bis 7, **dadurch gekennzeichnet, dass** der Schieber (16) im Bereich seines distalen Endes derart am Schaft (12) angelenkt ist, dass sein proximales Ende in der Freigabeposition vom Schaft (12) weggeschwenkt werden kann.

9. Schiebeschaftinstrument nach einem der Ansprüche 1 bis 8, **dadurch gekennzeichnet, dass** der Schaft (12) mit dem ersten Griffstück (14) eine erste gedachte Linie und der Schieber (16) mit dem zweiten Griffstücks (18) eine zweite gedachte Linie bilden, wobei sich die beiden Linien kreuzen, so dass bei natürlicher Griffhaltung das erste Griffstück (14) mit dem Daumen und das zweite Griffstück (18) mit dem Zeige- und/oder Mittelfinger gehalten werden.

10. Schiebeschaftinstrument nach einem der Ansprüche 1 bis 9, **dadurch gekennzeichnet, dass** der Schaft mit dem ersten Griffstück eine erste gedachte Linie und der Schieber mit dem zweiten Griffstücks eine zweite gedachte Linie bilden, wobei die beiden Linien einander nicht kreuzen, so dass bei natürlicher Griffhaltung das erste Griffstück mit dem Zeige- und/oder Mittelfinger und das zweite Griffstück mit dem Daumen gehalten werden.

## Claims

1. A surgical sliding shaft instrument (10) comprising
- a shaft (12), which proximally opens into a first handle piece (14), and
- a slide (16), which can be moved along the shaft (12) by means of a second handle piece (18) hinged to the first handle piece (14),
- wherein the slide (16) and the second handle piece (18) are detachably connected to one another via a locking mechanism,
- wherein the locking mechanism has a driver (36) and a mounting (34) and
- wherein the mounting (34) has a slot (40) for the driver (36) and the driver (36) can be moved into a working position, in which it is held in the mounting (34), and into a release position, in which it can exit the mounting (34) via the slot (40),
- wherein the driver (36) can be swivelled into the working position and into the release position by means of a lever (22),
- wherein the driver (36) can be locked in the working position,
- wherein the lever (22) is provided with a detent projection (52),
**characterized in**
- **that** the lever (22) in the working position of the driver (36) engages on one of the two handle pieces (18).

2. The sliding shaft instrument according to claim 1, **characterized in that** a guide channel (56) for the detent projection (52) is provided on the handle piece (18) on which the lever (22) engages in the working position of the driver (36).

3. The sliding shaft instrument according to claim 1 or 2, **characterized in that** a stop is provided for the lever (22).

4. The sliding shaft instrument according to claim 3, **characterized in that** the stop is formed by a hinge bolt (20), which connects the two handle pieces (14, 18) to one another in a hinged manner.

5. The sliding shaft instrument according to one of claims 1 to 4, **characterized in that** the mounting (34) is provided on the second handle piece (18) and that the driver (36) is guided by the slide (16) transversely to the longitudinal direction of the slide (16), wherein the slide (16) has in the region of the driver (36) a recess (42) for a lug (38) having the mounting (34).

6. The sliding shaft instrument according to one of claims 1 to 5, **characterized in that** the driver (36) has at least one narrow side (46) and at least one wide side (48), which are dimensioned such that the driver can only be guided with the narrow side (46) ahead through the slot (40).

7. The sliding shaft instrument according to claim 5 and claim 6, **characterized in that** the driver (36) is bordered at two ends, lying opposite one another, by two cylindrical end pieces (50), the external diameter of which is adapted to the internal diameter of a receiving bore provided in the slide (16).

8. The sliding shaft instrument according to one of claims 1 to 7, **characterized in that** the slide (16) is articuated in the region of its distal end on the shaft (12) such that its proximal end can be swivelled away from the shaft (12) in the release position.

9. The sliding shaft instrument according to one of claims 1 to 8, **characterized in that** the shaft (12) with the first handle piece (14) forms a first imaginary line and the the slide (16) with the second handle piece (18) forms a second imaginary line, wherein the two lines intersect, so that with a natural handle hold the first handle piece (14) is held by the thumb and the second handle piece (18) is held by the index and/or middle finger.

10. The sliding shaft instrument according to one of claims 1 to 9, **characterized in that** the shaft with the first handle piece forms a first imaginary line and the slide with the second handle piece forms a second imaginary line, wherein the two lines do not intersect, so that with a natural handle hold the first handle piece is held by the index and/or middle finger and the second handle piece is held by the thumb.

## Revendications

1. Instrument chirurgical à tige coulissante (10) comprenant
- une tige (12) qui débouche de façon proximale dans une première pièce de poignée (14), et
- un coulisseau (16) qui peut être déplacé le long de la tige (12) au moyen d'une deuxième pièce de poignée (18) reliée de manière articulée à la première pièce de poignée (14),
- dans lequel ledit coulisseau (16) et ladite deuxième pièce de poignée (18) sont reliés l'un à l'autre de manière amovible par l'intermédiaire d'un mécanisme de verrouillage,
- dans lequel le mécanisme de verrouillage comprend un entraîneur (36) et un logement (34), et
- dans lequel le logement (34) présente une fente (40) pour ledit entraîneur (36), et l'entraîneur (36) peut être déplacé dans une position de travail dans laquelle il est maintenu dans le logement (34), et dans une position de libération dans laquelle il peut quitter le logement (34) par ladite fente (40),
- dans lequel ledit entraîneur (36) peut être pivoté par le biais d'un levier (22) dans la position de travail et dans la position de libération,
- dans lequel l'entraîneur (36) pouvant être verrouillé dans la position de travail,
- dans lequel ledit levier (22) est pourvu d'une projection d'arrêt (52),
**caractérisé**
- **en ce que**, dans la position de travail de l'entraîneur (36), le levier (22) s'enclenche sur l'une des deux pièces de poignée (18).

2. Instrument à tige coulissante selon la revendication 1, **caractérisé en ce qu'**une rainure de guidage (56) pour la projection d'arrêt (52) est prévue sur la pièce de poignée (18) sur laquelle s'enclenche le levier (22) dans la position de travail de l'entraîneur (36).

3. Instrument à tige coulissante selon la revendication 1 ou 2, **caractérisé en ce qu'**une butée pour le levier (22) est prévue.

4. Instrument à tige coulissante selon la revendication 3, **caractérisé en ce que** ladite butée est constituée par un axe d'articulation (20) qui relie les deux pièces de poignée (14, 18) de manière articulée l'une à l'autre.

5. Instrument à tige coulissante selon l'une quelconque des revendications 1 à 4, **caractérisé en ce que** le logement (34) est prévu sur la deuxième pièce de poignée (18) et que l'entraîneur (36) passe à travers le coulisseau (16) transversalement à la direction longitudinale du coulisseau (16), dans lequel le coulisseau (16) possède, au niveau de l'entraîneur (36), un évidement (42) pour une languette (38) présentant le logement (34).

6. Instrument à tige coulissante selon l'une quelconque des revendications 1 à 5, **caractérisé en ce que** ledit entraîneur (36) présente au moins un côté étroit (46) et au moins un côté large (48) qui sont dimensionnés de telle sorte que l'on ne peut faire passer l'entraîneur à travers la fente (40) que le côté étroit (46) le premier.

7. Instrument à tige coulissante selon la revendication 5 et la revendication 6, **caractérisé en ce que** l'entraîneur (36) est entouré, à deux extrémités situées en regard l'une de l'autre, de deux pièces d'extrémité (50) cylindriques dont le diamètre extérieur est adapté au diamètre intérieur d'un trou de logement prévu dans le coulisseau (16).

8. Instrument à tige coulissante selon l'une quelconque des revendications 1 à 7, **caractérisé en ce que**, au niveau de son extrémité distale, le coulisseau (16) est articulé sur la tige (12) de telle sorte que, dans la position de libération, son extrémité proximale peut être pivotée dans la direction opposée à la tige (12).

9. Instrument à tige coulissante selon l'une quelconque des revendications 1 à 8, **caractérisé en ce que** la tige (12) forme avec la première pièce de poignée (14) une première ligne imaginaire et le coulisseau (16) forme avec la deuxième pièce de poignée (18) une deuxième ligne imaginaire, dans lequel les deux lignes se croisent de sorte que, lorsque la poignée est tenue de façon naturelle, la première pièce de poignée (14) est tenue par le pouce et la deuxième pièce de poignée (18) est tenue par l'index et/ou le majeur.

10. Instrument à tige coulissante selon l'une quelconque des revendications 1 à 9, **caractérisé en ce que** la tige forme avec la première pièce de poignée une première ligne imaginaire et le coulisseau forme avec la deuxième pièce de poignée une deuxième ligne imaginaire, dans lequel les deux lignes ne se croisent pas entre elles de sorte que, lorsque la poignée est tenue de façon naturelle, la première pièce de poignée est tenue par l'index et/ou le majeur et la deuxième pièce de poignée est tenue par le pouce.
